# EUROPEAN PATENT SPECIFICATION

(11) **EP 3 437 608 B1**
(45) Date of publication and mention of the grant of the patent: **24.02.2021**
(21) Application number: 17795888.1
(22) Date of filing: 14.04.2017
(51) Int. Cl.: A61F 13/511, A61F 13/514, A61L 15/20, A61L 15/48

(54) **ABSORBENT ARTICLE**
SAUGFÄHIGER ARTIKEL
ARTICLE ABSORBANT

(30) Priority: 13.05.2016 JP 2016097494
(43) Date of publication of application: 06.02.2019
(73) Proprietor: Unicharm Corporation, Ehime 799-0111 (JP)
(72) Inventor: MIYAMA, Takuya, Kanonji-shi Kagawa 769-1602 (JP); SAKAGUCHI, Satoru, Kanonji-shi Kagawa 769-1602 (JP); UDA, Masashi, Kanonji-shi Kagawa 769-1602 (JP)
(74) Representative: Dolleymores
(86) International application number: PCT/JP2017/015251
(87) International publication number: WO 2017/195527

(56) References cited:
- EP-A2- 1 166 731
- WO-A1-2015/082534
- JP-A- 2001 353 182
- JP-A- 2002 000 651
- JP-A- 2010 126 849
- JP-A- 2016 512 061
- JP-U- 3 154 532

## Description

### [TECHNICAL FIELD]

The present invention relates to an absorbent article provided with a nonwoven fabric including organic cotton fibers.

### [BACKGROUND ART]

Patent Literature 1 discloses an absorbent article provided with a nonwoven fabric including hydrophobic synthetic fibers and hydrophilic fibers. The hydrophilic fibers are natural cellulose fibers, such as cotton and rayon. At least a part of the hydrophilic fibers form aggregates that disperse in the sheet. At least a part of the hydrophilic fibers forming the aggregates (fiber lumps) is fused to the surface of the hydrophobic fibers. In addition, it has also been executed to impart hydrophilicity to the surface of the hydrophobic fibers by coating the surface with a surfactant or the like. Typically, a surfactant derived from petroleum is used in many cases.

### [CITATION LIST]

### [PATENT LITERATURE]

Patent Literature 1: JP 2002-651 A. EP1166731 A2 is a patent family member.

### [SUMMARY OF INVENTION]

In recent years, due to the growing awareness of protecting the natural environment and the growing awareness of desiring to use skin friendly materials, users are increasingly interested in naturally derived materials that have been cultivated without agricultural chemicals. In particular, there is an increasing need to use skin-friendly materials for absorbent articles such as disposable diapers used for infants. From this point of view, the inventor of the present application considers taking organic cotton fibers as the hydrophilic fibers (natural cellulose fibers) included in the absorbent article.

Compared to hydrophobic synthetic fibers, cotton fibers tend to absorb moisture, so that the cotton fibers act to draw moisture from the area around the cotton fibers into the cotton fiber lumps. When a certain amount of moisture is retained in the cotton fiber lumps, some of the moisture is released to the outside.

Here, if there is a non-natural substance such as a petroleum-derived oil agent on the surfaces of the cotton fibers or around the cotton fibers, there is a concern that the non-natural substance seeps out into the moisture in the cotton fiber lumps.

Even when the organic cotton fibers are used as the cotton fibers, if the non-natural substance seeps out into the moisture retained in the fiber lumps, users might not have a sense of security guaranteeing gentleness to the skin.

It is, therefore, desired to provide an absorbent article capable of giving a sense of security to users.

The present invention provides the absorbent article of independent claim 1. The dependent claims specify preferred but optional features.

### [BRIEF DESCRIPTION OF DRAWINGS]

Fig. 1 is a plan view of an absorbent article according to an embodiment.
Fig. 2 is a plan view of the absorbent article when seen from the opposite side of Fig. 1.
Fig. 3 is a schematic view illustrating a cross section of a topsheet or a backsheet.
Fig. 4 is a schematic view illustrating a cross section of a part of an absorbent article.
Fig. 5 is a schematic diagram illustrating process steps of a method of manufacturing a nonwoven fabric according to the embodiment.
Fig. 6 is a schematic diagram illustrating process steps following Fig. 5.

### [DESCRIPTION OF EMBODIMENTS]

According to the present specification and the accompanying drawings, at least the following matters will be disclosed.

An absorbent article including a nonwoven fabric, in which the nonwoven fabric includes organic cotton fibers and thermoplastic synthetic fibers, the organic cotton fibers forming a plurality of fiber lumps in the nonwoven fabric, and the surfaces of the organic cotton fibers are not coated with a petroleum-derived oil agent.

The organic cotton fibers are cultivated with no pesticide or low pesticide, so that it is possible to give the user a sense of security guaranteeing gentleness to the global environment as well as to the skin. Also, the surfaces of the organic cotton fibers are not coated with the petroleum-derived oil agent. Therefore, even when the fiber lumps made of organic cotton fibers retain moisture, it is possible to decrease the sense of uneasiness that petroleum-derived oil agent seeps out in the moisture. Therefore, it is possible to give the user a sense of security guaranteeing gentleness to the skin.

According to a preferred embodiment, the synthetic fibers are coated with a plant-derived oil agent.

The surfaces of the synthetic fibers are coated with a plant-derived oil agent, instead of a petroleum-derived oil agent. Thus, it is possible to impart hydrophilicity or water repellency to the synthetic fibers depending on the purpose. When the organic cotton fibers absorb moisture from their surroundings, the oil agent coated on the surfaces of the synthetic fibers may also be drawn. Since the oil agent coated on the surfaces of the synthetic fibers is a plant-derived oil agent, the plant-derived oil, not a petroleum-derived oil, is mixed in the moisture retained in the organic cotton fibers. Therefore, it is possible to give the user a sense of security guaranteeing gentleness to the skin.

According to a preferred embodiment, the surfaces of the fiber lumps are coated with a plant-derived oil agent.

The surfaces of the fiber lumps of the organic cotton fibers are coated with a plant-derived oil agent, instead of a petroleum-derived oil agent. Therefore, even when the oil agent oozes out into the moisture retained in the organic cotton fibers, the oil agent mixed in the moisture is derived from plants. Therefore, it is possible to give the user a sense of security guaranteeing gentleness to the skin.

According to a preferred embodiment, a ratio of the weight of the organic cotton fibers is relative to the weight of the whole nonwoven fabric is in the range of 2% to 50%. The organic cotton fibers absorb moisture from their surroundings and release the absorbed moisture to the outside. Thus, the organic cotton fibers act to balance the absorption and release of moisture. Since the weight of the organic cotton fibers is 2% to 50% of the weight of the whole nonwoven fabric, it is possible to balance water absorption and release of the whole nonwoven fabric.

According to a preferred embodiment, a region having a density lower than the density of the fiber lumps and the synthetic fibers is formed around individual fiber lumps. When the fiber lumps made of organic cotton fibers absorb moisture, the fiber lumps inflate. At this time, since the low density region exists around the individual fiber lumps, the fiber lumps can inflate without being strongly pressed from the surroundings. Therefore, the fiber lumps can more effectively absorb moisture.

According to a preferred embodiment, the organic cotton fibers are not thermally fused to the synthetic fibers. Since the organic cotton fibers are not thermally fused to the synthetic fibers, migration of the oil agent from the synthetic fibers to the organic cotton fibers can be decreased even if the synthetic fibers are coated with a petroleum-derived oil agent. As a result, it is possible to give the user a sense of security guaranteeing gentleness to the skin.

According to a preferred embodiment, the plurality of fiber lumps are provided biased toward the center portion of the nonwoven fabric in the thickness direction of the nonwoven fabric. Since the fiber lumps made of organic cotton fibers that easily absorb moisture are provided in the center portion of the nonwoven fabric in the thickness direction, it is possible to keep the surface of the nonwoven fabric as dry as possible. Thus, it is possible to provide the user an absorbent article which is gentle to the skin.

According to the present invention, the absorbent article includes a front-back direction, a width direction which is orthogonal to the front-back direction, a topsheet, a backsheet, an absorber provided between the topsheet and the backsheet, and a liquid-impermeable leakage-preventing sheet provided between the backsheet and the absorber and covering the absorber, in which at least one of the topsheet and the backsheet is made of the nonwoven fabric, the nonwoven fabric includes a region not overlapping the leakage-preventing sheet, and the region of the nonwoven fabric not overlapping the leakage-preventing sheet extends at least from the front side to the rear side of the absorbent article on the outside in the width direction. That is, the region corresponding to the leg openings of the wearer does not include the leakage-preventing sheet and is made of the nonwoven fabric. The fiber lumps in the nonwoven fabric act to absorb and release moisture. Therefore, the region of the leg openings of the absorbent article releases moisture absorbed from the side of the skin of the wearer to the outside. As a result, it is possible to keep the region of the leg openings of the absorbent article as dry as possible.

According to a preferred embodiment, the organic cotton fibers have an average fiber length shorter than the average fiber length of the synthetic fibers. By increasing the average fiber length of synthetic fibers, the strength of the entire nonwoven fabric can be improved, and it is possible to decrease fuzzing of the fibers during manufacturing and during use. On the other hand, by decreasing the average fiber length of the organic cotton fibers, it is possible to decrease the thermal fusing point between the synthetic fibers and realize appropriate flexibility as the nonwoven fabric.

Hereinafter, an absorbent article according to an embodiment will be described in detail by referring to the accompanying drawings. The absorbent article of the present invention is, for example, a disposable diaper or a sanitary napkin. In the following embodiment, a disposable diaper is taken as an example and described in detail.

In the drawings, the same or similar parts are indicated by the same or similar reference signs. The drawings, however, are illustrated schematically, and dimensional ratios and other variables differ from those of actual measurements. The actual measurements or the like, therefore, should be determined by referring to the following description. The drawings may include different relationships or ratios of measurements.

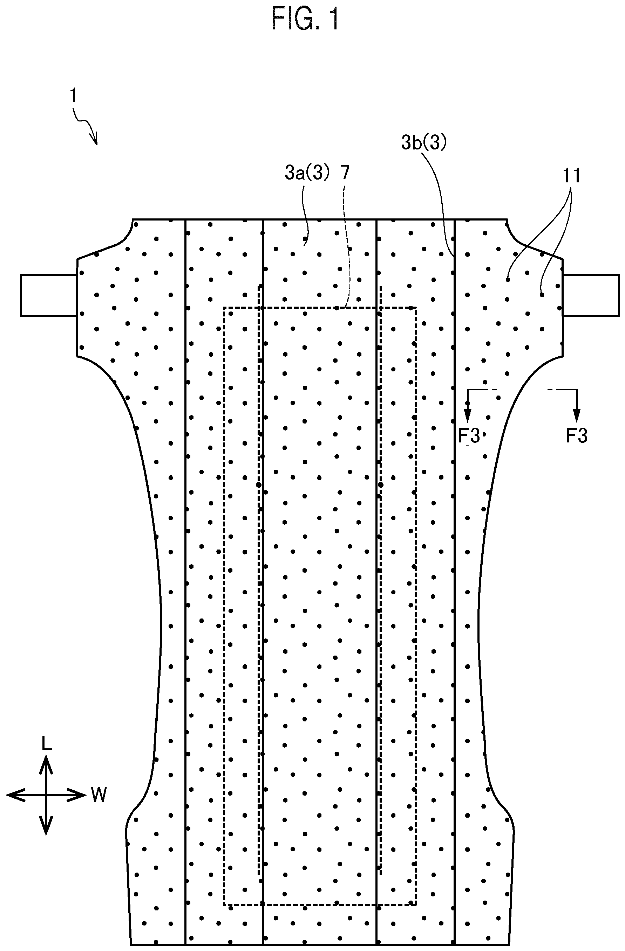

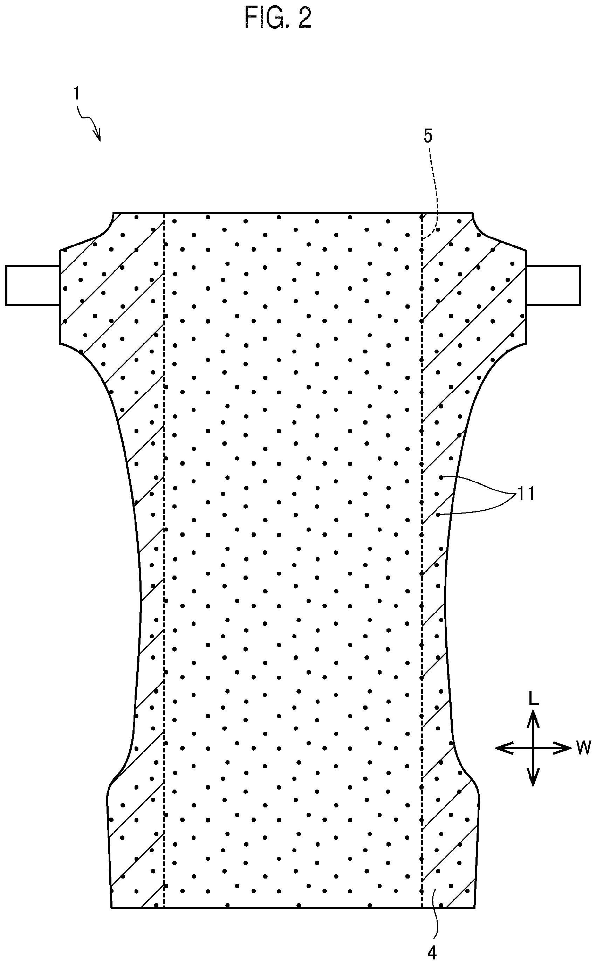

Fig. 1 is a plan view of an absorbent article 1 according to an embodiment. Fig. 2 is a plan view of the absorbent article 1 when seen from the opposite side of Fig. 1. The absorbent article 1 includes a longitudinal direction L and a width direction W. The longitudinal direction L extends from the front side (ventral side) toward the rear side (dorsal side) of the wearer, or from the rear side toward the front side of the wearer. The width direction W is a direction orthogonal to the longitudinal direction L. Hereinafter, the side facing the skin of the wearer during use may be referred to as a "skin surface side". In addition, the side opposite to the skin of the wearer during use may be referred to as a "non-skin surface side".

The absorbent article 1 includes a topsheet 3, a backsheet 4, and an absorber 7. The absorber 7 is disposed between the topsheet 3 and the backsheet 4. The topsheet 3 may be made of a liquid-permeable sheet.

A liquid-impermeable leakage-preventing sheet 5 is provided between the backsheet 4 and the absorber 7. The leakage-preventing sheet 5 at least covers the absorber 7. The backsheet 4 covers the leakage-preventing sheet 5 and extends to the outside of the leakage-preventing sheet 5. A region of the backsheet 4 not overlapping the leakage-preventing sheet 5 extends from the front end to the rear end in the longitudinal direction L on both outer sides in the width direction W. In Fig. 2, a region of the backsheet 4 not overlapping the leakage-preventing sheet 5 is indicated by hatched lines.

The topsheet 3 may include a liquid-permeable sheet 3a covering the absorber 7, and a side sheet 3b covering an outer portion of the liquid-permeable sheet 3a in the width direction W and extending toward the outside of the liquid-permeable sheet 3a. The inner edge of the side sheet 3b in the width direction W is configured to rise up and may be formed as a leakage-preventing gather.

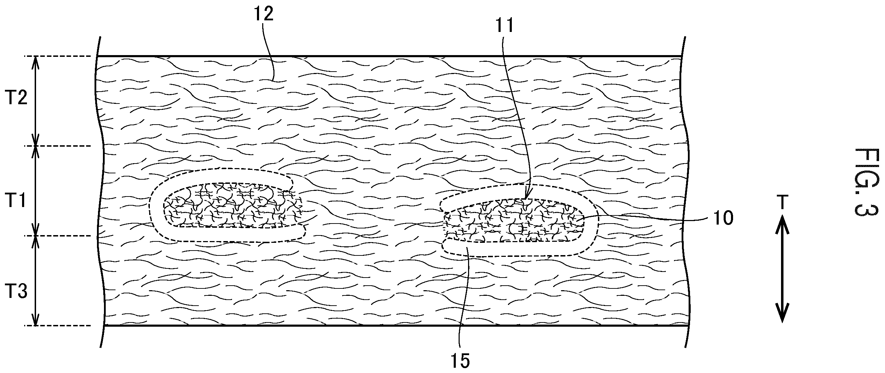

The topsheet 3 and the backsheet 4 are made of a nonwoven fabric. Fig. 3 is a schematic view illustrating a cross section of the topsheet 3 or the backsheet 4. The nonwoven fabric includes organic cotton fibers 10 and synthetic fibers 12. As used herein, "organic cotton" refers to cotton that is certified organic and is so certified in accordance with international standards (standards of each country conforming to CODEX) at the agricultural stage.

The synthetic fibers 12 may be fibers made of a thermoplastic resin. Examples of fibers made of the thermoplastic resin include an olefin resin, a polyester resin, a polyamide resin, and any combination of these resins. The fibers made of the thermoplastic resin may be coated with an oil agent. The fibers made of the thermoplastic resin may be made hydrophilic with a hydrophilizing oil agent or may have water repellency with a water repellent oil agent, depending on the purpose. For example, in a case where the nonwoven fabric is used as the liquid-permeable sheet 3a of the topsheet 3, it is preferable to coat the synthetic fibers 12 with a hydrophilizing oil agent. In a case where the nonwoven fabric is used as the side sheet 3b of the backsheet 4 or the topsheet 3, it is preferable to coat the synthetic fibers 12 with a water repellent oil agent. Furthermore, in a case where the nonwoven fabric is used as the side sheet 3b of the topsheet 3, it is more preferable to coat the synthetic fibers 12 with a water repellent oil agent having higher water repellency than the nonwoven fabric as the backsheet 4.

The organic cotton fibers 10 form a plurality of fiber lumps 11 in the nonwoven fabric. The organic cotton fibers 10 may have an average fiber length shorter than that of the synthetic fibers 12. As a result, the organic cotton fibers 10 can more easily form the fiber lumps 11 in the nonwoven fabric.

The surfaces of the fiber lumps 11 of the organic cotton fibers 10 are not coated with a petroleum-derived oil agent. Since the organic cotton fibers 10 are cultivated with no pesticide or low pesticide, it is possible to give the user a sense of security guaranteeing gentleness to the skin. In addition, the surfaces of the organic cotton fibers 10 are not coated with a petroleum-derived oil agent. Therefore, even when the fiber lumps 11 made of the organic cotton fibers 10 retain moisture, it is possible to decrease the sense of uneasiness that the petroleum-derived oil agent oozes out into the moisture. Therefore, it is possible to give the user a sense of security guaranteeing gentleness to the skin.

Alternatively, the surfaces of the fiber lumps 11 of the organic cotton fibers 10 may be coated with a plant-derived oil agent. For example, by coating the synthetic fibers 12 around the fiber lumps 11 with the oil agent, the surface of the fiber lumps 11 can be coated (covered) with the plant-derived oil agent, which will be described later.

In addition, in the case where the surfaces of the organic cotton fibers 10 are coated with the plant-derived oil agent, even when the oil agent oozes out into the moisture retained by the organic cotton fibers, the oil agent mixed in the moisture is derived from a plant. Therefore, it is possible to give the user a sense of security guaranteeing gentleness to the skin.

The oil agent to be coated on the synthetic fibers 12 may be petroleum-derived or plant-derived. The oil agent can impart hydrophilicity or water repellency to the synthetic fiber 12.

The oil agent is preferably plant-derived. When the organic cotton fibers 10 absorb moisture from the surroundings, the oil agent coated on the surfaces of the synthetic fibers 12 may also be drawn. Since the oil agent coated on the surface of the synthetic fibers 12 is the plant-derived oil agent, the plant-derived oil agent, not the petroleum-derived oil agent, is mixed in the moisture retained in the organic cotton fibers 10. Therefore, it is possible to give the user a sense of security guaranteeing gentleness to the skin.

Examples of the plant-derived oil agent coated on the synthetic fibers 12 include, for example, an oil agent derived from a component extracted from rice oil, coconut oil, camellia oil, tea leaves or eucalyptus, an oil agent derived from a component extracted from rice bran, or an oil agent derived from a combination of these components. From the viewpoint of the sense of security, it is more preferable that the plant-derived oil agent is derived from the component extracted from something that can be used as food.

Also, the oil agent is preferably weakly acidic. By using an oil agent having a pH close to human skin, a nonwoven fabric can be gentle to the skin.

A ratio of the weight of the organic cotton fibers 10 to the weight of the whole nonwoven fabric is in the range of 2% to 50%, preferably 3% to 35%, and more preferably 3% to 10%. Organic cotton fibers absorb moisture from the surroundings and release absorbed moisture to the outside. As described above, the organic cotton fibers 10 act to balance the absorption and release of moisture. With the above range of the weight of the organic cotton fibers 10 relative to the weight of the entire nonwoven fabric, it is possible to balance the absorption and release of moisture of the entire nonwoven fabric.

A fineness of the synthetic fibers 12 is not particularly limited, but may be in the range of 1.1 dtex to 8.8 dtex, preferably 1.5 dtex to 4.6 dtex, from the viewpoints of the strength, flexibility, feel of touch, liquid permeability and the like of the nonwoven fabric.

Alternatively, the synthetic fibers may include preferably 10% to 40%, more preferably 20% to 30%, of fibers having the fineness of less than 2.0 dtex in the fibers having the fineness of at least 2.0 dtex. In this case, the synthetic fibers having a short fiber length can be included, while maintaining a long average fiber length. If the synthetic fibers 12 include thin count fibers, it is possible to further decrease the fiber lumps 11 of the organic cotton fibers 10 from falling off from the nonwoven fabric.

Preferably, as illustrated in Fig. 3, the plurality of fiber lumps 11 are disposed biased to the center side of the absorbent article 1 in the thickness direction T. It is assumed herein that, if the absorbent article 1 is equally divided into three parts including two layers T2 and T3 in the thickness direction T on the surface sides and a middle layer T1, and at least 60%, preferably at least 70%, of the plurality of fiber lumps 11 exists in the center layer T1, the fiber lumps 11 are provided biased to the center of the absorbent article 1 in the thickness direction T.

Since the fiber lumps 11 made of the organic cotton fibers 10 which easily absorb moisture are disposed in the center of the nonwoven fabric in the thickness direction T, it is possible to keep the surface of the nonwoven fabric as dry as possible. Thus, it is possible to provide the absorbent article 1 with good touch to the user.

In order to confirm the biasing of the fiber lumps 11 in the thickness direction, X-ray CT can be used. Specifically, an X-ray CT (e.g., FLEX-M863 manufactured by BEAMSENSE Co., Ltd.) is used to photograph the nonwoven fabric including a portion of the fiber lump 11 to be measured from one side to the other side of the nonwoven fabric. Using the image obtained by photographing, it is possible to determine the biasing of the fiber lumps 11 by checking ten fiber lumps 11 in the same nonwoven fabric. It is possible to confirm whether the fiber lumps 11 in the sample after photographing are the fiber lumps 11 of organic cotton fibers by a dyeing method with KayastainQ, which will be described later.

Typically, the nonwoven fabric preferably has a basis weight in the range of 10 g/cm² to 60 g/cm². The thickness of the nonwoven fabric is preferably 0.5 mm to 5.0 mm, and more preferably 0.8 mm to 3.5 mm. The thickness of the nonwoven fabric can be measured using a thickness meter (Peacock dial thickness gauge manufactured by OZAKI MFG. CO., LTD.) having a measuring terminal with a measuring pressure of 3g/cm² and a diameter of 10 mm. Specifically, the thickness is measured at ten different points of the target nonwoven fabric, and the "thickness" is obtained from the average value.

In the nonwoven fabric having the above thickness, it is preferable to set the weight ratio of organic cotton fibers to synthetic fibers to less than 25%. Under this condition, it is possible to further decrease the falling off of the organic cotton fibers during manufacturing and during use by disposing the fiber lumps 11 biased to the center of the nonwoven fabric in the thickness direction.

The weight ratio of the organic cotton fibers to the synthetic fibers can be calculated by the following method. First, a test piece of a unit area (e.g., 5 cm × 5 cm) is taken out from the nonwoven fabric. The organic cotton fibers are taken out from the test piece, and the weight of the taken-out organic cotton fibers is measured. As a result, the weight ratio of the organic cotton fibers to the synthetic fibers can be calculated.

In addition, in extracting the organic cotton fibers from the test piece, it is necessary to first discriminate the difference between synthetic fibers and organic cotton fibers. Whether the fibers in the nonwoven fabric are made of organic cotton fibers or synthetic fibers can be confirmed by the following method. First, an amount of KayastainQ (manufactured by Nippon Kayaku Co., Ltd.) equivalent to the amount of the test piece is weighed and dissolved in ion exchanged water (60 to 70 degrees) having a weight 100 times heavier the weight of KayastainQ. This solution is heated and, just before the solution boils, the test piece that has been washed with a neutral detergent, put into the solution. After the solution was boiled for 5 minutes, the test piece was rapidly washed with water and dried. At this point, the fiber lumps are dyed in colors according to the types of fibers. Subsequently, the fiber lumps can be discriminated on the basis of the hues of KayastainQ for various fibers.

The organic cotton fibers 10 included in the nonwoven fabric preferably have an average fiber length shorter than the average fiber length of the synthetic fibers 12 and/or have a fineness smaller than the fineness of the synthetic fibers 12. By increasing the average fiber length of the synthetic fibers 12 and/or increasing the fineness of the synthetic fibers 12, it is possible to improve the strength of the entire nonwoven fabric and decrease fuzzing of the fibers during manufacturing and during use. On the other hand, by decreasing the average fiber length of the organic cotton fibers 10 and/or decreasing the fineness of the organic cotton fibers 10, it is possible to decrease the thermal fusing point among the synthetic fibers 12 and achieve moderate flexibility as a nonwoven fabric.

It is assumed that the "average fiber length" refers to the average fiber length measured according to "A7.1.1 A method (standard method): A method of measuring the length of individual fibers on a glass plate with scale" in "A7.1 Measurement of fiber length" of Annex A, JIS L 1015: 2010. The above method corresponds to the test method equivalent to ISO 6989 issued in 1981.

Fig. 3 is a cross section of a single layer nonwoven fabric constituting the topsheet 3 or the backsheet 4. Alternatively, the topsheet 3 or the backsheet 4 may have a multilayered structure in which a plurality of nonwoven fabrics are layered.

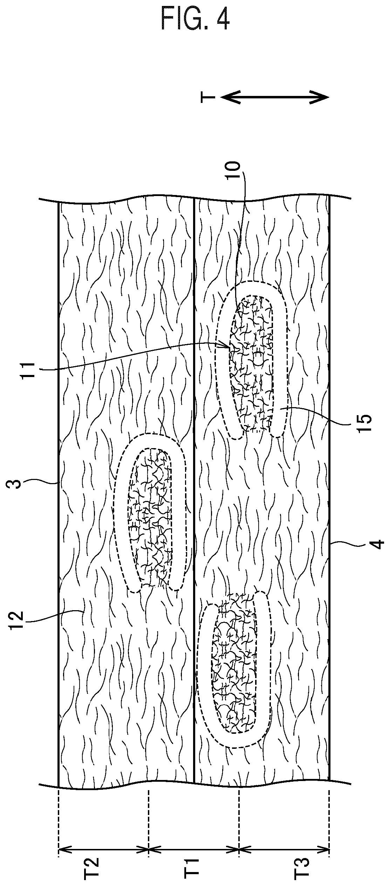

Fig. 4 illustrates a cross section of a part of the absorbent article (e.g., along line F3-F3 of Fig. 1). In this region, the nonwoven fabric constituting the topsheet 3 and the nonwoven fabric constituting the backsheet 4 overlap each other. More specifically, at the position of line F3-F3 of Fig. 1, the side sheet 3b and the backsheet 4 overlap each other.

In this case, the fiber lumps 11 of the organic cotton fibers 10 need to be arranged biased toward the center in the thickness direction T of the absorbent article 1. Specifically, even when the fiber lumps 11 are arranged biased on the surface of the individual topsheets 3 and the backsheet 4, the entire topsheet 3 and the backsheet 4 as a whole in the thickness direction T of the absorbent article 1 need to be arranged biased toward the center. Thus, the surface of the absorbent article can be kept as dry as possible, and it is possible to provide the absorbent article 1 with good touch to the user.

Preferably, as illustrated in Figs. 3 and 4, a region 15 having a density smaller than the density of the fiber lump 11 and the synthetic fibers 12 is formed around the fiber lump 11 made of the organic cotton fibers 10. When the fiber lumps 11 made of the organic cotton fibers 10 absorb moisture, the fiber lumps 11 inflate. At this time, since there is a region with a low density around the fiber lump 11, the fiber lumps 11 can inflate without being strongly pressed from the surroundings. Therefore, the fiber lumps 11 can more effectively absorb moisture. As a result, the moisturizing effect of the organic cotton fibers 10 can be sufficiently exerted.

Preferably, the organic cotton fibers 10 are not thermally fused to the synthetic fibers 12. Since the organic cotton fibers 10 are not thermally fused to the synthetic fibers 12, the migration of the oil agent from the synthetic fibers 12 to the organic cotton fibers 10 can be decreased. As a result, it is possible to give the user a sense of security guaranteeing gentleness to the skin.

It is preferable that the organic cotton fibers 10 are included in at least the nonwoven fabric in a region not overlapping with the leakage-preventing sheet 5 among the nonwoven fabrics constituting the absorbent article 1. Specifically, of the topsheet 3 and/or the backsheet 4, which is/are made of the nonwoven fabric including the organic cotton fibers 10 described above, the region that does not overlap the leakage-preventing sheet 5 is at least located outside in the width direction W to at least extend from the front side to the rear side of the absorbent article 1. That is, the region corresponding to the leg-openings of the wearer does not include the leakage-preventing sheet 5 and is made only of the nonwoven fabric including the organic cotton fibers 10. The fiber lumps 11 in the nonwoven fabric act to absorb and release moisture. Therefore, the leg-opening regions of the absorbent article 1 release moisture absorbed from the skin of the wearer to the outside. As a result, it is possible to keep the region around the legs of the absorbent article 1 as dry as possible.

The liquid-permeable sheet 3a constituting the topsheet 3 is preferably made of a nonwoven fabric including the organic cotton fibers 10 as described above. In this case, the synthetic fibers 12 constituting the nonwoven fabric are preferably coated with a hydrophilizing oil agent. The liquid-permeable sheet 3a is disposed at a position corresponding to the crotch of the wearer and has a large area that is in contact with the skin of the wearer. Therefore, by using the nonwoven fabric including the organic cotton fibers 10 as the liquid-permeable sheet 3a, it is possible to give the user a sense of security guaranteeing gentleness to the skin.

Further, since the backsheet 3b is a portion where the user can easily touch from the outside, it is also possible to give the user a sense of security guaranteeing gentleness to the skin when the nonwoven fabric including the organic cotton fibers 10 is used as the backsheet 3b.

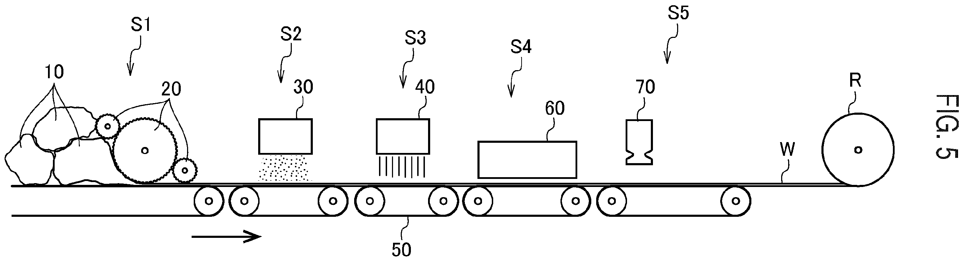

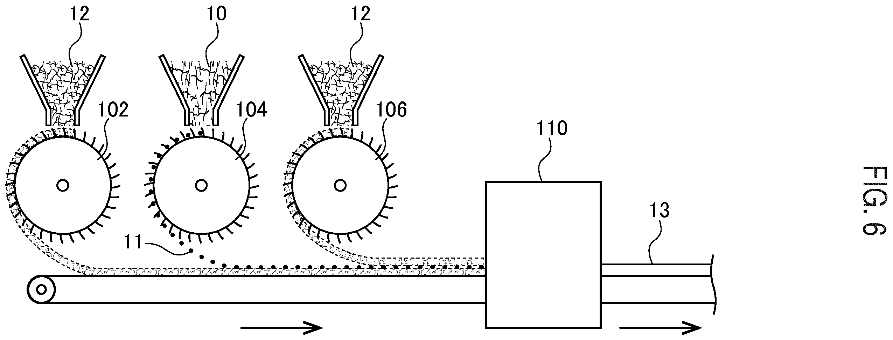

Fig. 5 is a schematic diagram illustrating an example method of manufacturing the nonwoven fabric including the fiber lumps 11 made of the organic cotton fibers 10. Fig. 6 is a schematic diagram illustrating the process steps following Fig. 1.

First, raw cotton of the organic cotton fibers 10 is prepared. Next, the raw cotton of the organic cotton fibers 10 is formed into a web-like shape (step S1). For example, the raw cotton of the organic cotton fibers 10 can be formed into a web-like shape by a carding apparatus 20. The organic cotton fibers 10 in the web-like shape are conveyed on a conveyor belt.

Next, the web of the organic cotton fibers 10 is preferably made wet before step S3 described later (step S2). Step S2 is carried out optionally. Humidification of the organic cotton fibers 10 can be carried out, for example, by a humidifier 30 that sprays water droplets. By wetting the web of the organic cotton fibers 10 before spraying a high-pressure water flow to the web in step S3, a sheet having a more uniform fiber density can be provided.

Next, the web of the organic cotton fiber 10 is placed on a mesh 50, and water is sprayed onto the web of the organic cotton fibers 10 to form a sheet (step S3). The water flow sprayed onto the web of the organic cotton fibers 10 is sprayed by the spraying apparatus 40 at a high water pressure from nozzles having a small nozzle diameter.

As a result, the organic cotton fibers 10 having a short fiber length are removed through the openings of the mesh 50. In addition, by spraying the water flow, fine foreign objects are also removed from the openings of the mesh 50. Therefore, it is possible to decrease falling off of the organic cotton fibers 10 having a small fiber length in the steps from the step S3 (in particular, the step of forming the nonwoven fabric from the mixed fibers). As a result, it is possible to decrease the adhesion of fine powder to various facilities disposed during the manufacturing process steps.

Further, in the step S3, by spraying the water flow to the web of the organic cotton fibers 10, the organic cotton fibers 10 move so as to be separated from the hard foreign object, whereby the foreign object can easily appear on the surface of the organic cotton fibers 10. Therefore, the foreign object can be made evident on the surface of the sheet of the organic cotton fibers 10. Thus, the foreign object can be removed more reliably.

The opening ratio of the mesh is preferably in the range of 5% to 40%, and more preferably in the range of 5% to 20%. This makes it possible to effectively remove the organic cotton fibers having short fiber lengths and easily falling off as fine powder during the manufacturing process steps. The opening diameter of the mesh is preferably 0.2 mm to 0.6 mm, and more preferably 0.25 mm to 0.45 mm.

In Fig. 1, the water flow is sprayed from one side of the sheet of the organic cotton fibers 10 to the web of the organic cotton fibers 10. The water flow sprayed onto the web of the organic cotton fibers 10 can expose the foreign object on the sheet by pushing out the fibers around the foreign object. As a result, it is possible to more effectively expose the foreign substance on the surface of the sheet. Preferably, the pressure of the water flow sprayed onto the sheet of the organic cotton fibers 10 is adjusted appropriately to make the foreign object be exposed more easily on the surface of the sheet.

Next, the sheet of the organic cotton fibers 10 is dried (step S4). The sheet of the organic cotton fibers 10 is dried using a dryer 60.

Next, the foreign object appearing on the surface of the sheet of the organic cotton fiber 10 is removed (step S5). Specifically, in one example, after spraying the water flow on the web of the organic cotton fibers 10 and before spreading a sheet of the organic cotton fibers, which will be described later, the foreign object exposed on the surface of the organic cotton fibers 10 is removed first. The position of the foreign object may be automatically specified by a camera 70. In step S3, since the foreign object appears on the surface of the sheet of the organic cotton fibers 10, the foreign object can be removed more reliably.

A sheet W of the organic cotton fibers 10 from which the foreign object has been removed may be wound on a roll R at one end.

Next, the sheet of organic cotton fibers is opened to form cotton-like organic cotton fibers. It should be noted that the organic cotton fibers with short fiber lengths have been largely removed by steps S1 to S5 described above. In particular, it is preferable that, by adjusting the pressure of the water flow and the floor efficiency of the mesh in step S3, the number of the organic cotton fibers 10 having a fiber length of less than 0.3 mm is adjusted to not exceed 1% of the number of the organic cotton fibers 10 having a fiber length of at least 0.3 mm. This makes it possible to decrease the falling off of the organic cotton fibers during the manufacturing process steps of the nonwoven fabric. In addition, it is possible to reduce particularly fine fibers in the organic cotton fibers, that is, fibers which are liable to fall off from the gaps between the fibers of the nonwoven fabric, and to decrease generation of fine powder during the manufacturing process steps. Furthermore, in the case where the organic cotton fibers are used in the absorbent article, it is possible to decrease a decrease in sheet-to-sheet joining strength due to dropped fine fibers, whereby moldability and strength of the absorbent article can be ensured during use.

Next, a nonwoven fabric is formed from mixed fibers of synthetic fibers 12 and cotton-like organic cotton fibers 10. The synthetic fibers 12 may be fibers made of a thermoplastic resin. Examples of fibers made of the thermoplastic resin include an olefin resin, a polyester resin, a polyamide resin, and any combination of these resins. The fibers made of a thermoplastic resin may be coated with the above-mentioned oil agent. Preferably, coating timing of the oil agent is before the synthetic fibers 12 are mixed with the organic cotton fibers 10.

An example of a method for forming a nonwoven fabric from mixed fibers will be described with reference to Fig. 6. First, the synthetic fibers 12 are supplied from a first-stage carding device 102. Further, the organic cotton fibers 10 that have undergone the above steps S1 to S5 are supplied from a second-stage carding device 104. Since the organic cotton fibers 10 is a low rigidity and soft material, it can be made into lump-shaped fiber lumps 11 with a pin of the carding device 104. Further, the synthetic fibers 12 are supplied from a third-stage carding device 106. As a result, the mixed fibers in which the fiber lumps 11 made of organic cotton fibers are arranged biased toward the center in the thickness direction. Accordingly, it is possible to decrease the fiber lumps 11 from falling off from the mixed fibers.

The nonwoven fabric 13 is manufactured by feeding the mixed fibers to a heating chamber 110 of, for example, a through air system and thermally fusing the synthetic fibers 12. In addition, the organic cotton fibers 10 are not thermally fused, but are left in the nonwoven fabric 13 due to entanglement of the fibers.

A ratio of the weight of the organic cotton fibers 10 to the weight of the whole mixed fibers, that is, the weight of the entire nonwoven fabric is 2% to 50%, preferably 3% to 35%, and more preferably 3% to 10%. If there is a large amount of the organic cotton fibers, the fiber lumps 11 absorb a large amount of moisture, giving discomfort to the wearer wearing the absorbent article.

A fineness of the synthetic fibers 12 is not particularly limited, but may be in the range of 1.1 dtex to 8.8 dtex, preferably 1.5 dtex to 4.6 dtex, from the viewpoints of the strength, flexibility, feel of touch, liquid permeability and the like of the nonwoven fabric.

Alternatively, the synthetic fibers may include preferably 10% to 40%, and more preferably 20% to 30% of fibers having the fineness of less than 2.0 dtex among the fibers having the fineness of at least 2.0 dtex. In this case, the synthetic fibers having a short fiber length can be included, while maintaining a long average fiber length. When the synthetic fibers 12 contain thin count fibers, it is possible to further decrease the fiber lumps of the organic cotton fibers from falling off from the nonwoven fabric.

Although the embodiment of the present invention has been described above in detail, it is apparent for persons who have ordinary skill in the art that the embodiment described in this specification does not limit the scope of the present invention. Changes and modifications may apply to the embodiment of the present invention within the scope of the present invention that is defined by the description of the appended claims. The description of the present specification, therefore, has been understood to be illustrative and is in no way intended to limit the scope of the invention.

For example, the embodiment described above has illustrated a method of manufacturing a nonwoven fabric using a through-air type heating chamber. Alternatively, any other known method may be employed for producing nonwoven fabrics from mixed fibers.

## Claims

1. An absorbent article (1), comprising:
a nonwoven fabric, wherein
the nonwoven fabric includes organic cotton fibers (10) and thermoplastic synthetic fibers (12),
the organic cotton fibers form a plurality of fiber lumps (11) in the nonwoven fabric, and
surfaces of the organic cotton fibers are not coated with a petroleum-derived oil agent, and wherein
the absorbent article (1) includes
a longitudinal direction (L),
a width direction (W) orthogonal to the longitudinal direction,
a topsheet (3),
a backsheet (4) ,
an absorber (7) between the topsheet and the backsheet, and
a liquid-impermeable leakage-preventing sheet (5) provided between the backsheet and the absorber and
covering the absorber,
at least one of the topsheet and the backsheet is made of the nonwoven fabric,
the nonwoven fabric has a region not overlapping the leakage-preventing sheet (5), and
the region of the nonwoven fabric not overlapping the leakage-preventing sheet extends at least from the front side to the rear side of the absorbent article on the outside in the width direction.

2. The absorbent article (1) according to claim 1, wherein
a region (15) having a density lower than a density of each of the fiber lumps (11) and a density of the synthetic fibers (12) is formed around individual fiber lumps.

3. The absorbent article (1) according to claim 1 or claim 2, wherein
the organic cotton fibers (10) are not thermally fused to the synthetic fibers (12).

4. The absorbent article according to any of claims 1 to 3, wherein
the surface of each of the fiber lumps (11) is coated with the plant-derived oil agent.

5. The absorbent article according to any one of claims 1 to 4, wherein
a ratio of a weight of the organic cotton fibers (10) relative to a total weight of the nonwoven fabric is in the range of 2% to 50%.

6. The absorbent article according to any one of claims 1 to 5, wherein
the organic cotton fibers (10) have an average fiber length shorter than the average fiber length of the synthetic fibers (12).

7. The absorbent article according to any one of claims 1 to 6, wherein the synthetic fibers are coated with a plant-derived oil agent.

8. The absorbent article according to any one of claims 1 to 7, wherein the plurality of fiber lumps is provided biased toward a center portion of the nonwoven fabric in a thickness direction of the nonwoven fabric.

## Patentansprüche

1. Absorbierender Artikel (1), der Folgendes umfasst:
einen Vliesstoff, wobei
der Vliesstoff Biobaumwollfasern (10) und thermoplastische synthetische Fasern (12) einschließt,
die Biobaumwollfasern eine Vielzahl von Faserklumpen (11) in dem Vliesstoff bilden und
Oberflächen der Biobaumwollfasern nicht mit einem aus Erdöl stammenden Ölmittel beschichtet sind und wobei der absorbierende Artikel (1) Folgendes einschließt:
eine Längsrichtung (L),
eine Breitenrichtung (W) rechtwinklig zu der Längsrichtung,
eine obere Lage (3),
eine hintere Lage (4),
einen Absorber (7) zwischen der oberen Lage und der hinteren Lage und
eine flüssigkeitsundurchlässige, ein Auslaufen verhindernde Lage (5), die zwischen der hinteren Lage und dem Absorber bereitgestellt ist und den Absorber abdeckt,
mindestens eine der oberen Lage und der hinteren Lage aus dem Vliesstoff hergestellt ist,
der Vliesstoff einen Bereich aufweist, der nicht mit der ein Auslaufen verhindernden Lage (5) überlappt und
sich der Bereich des Vliesstoffs, der nicht mit der ein Auslaufen verhindernden Lage überlappt, mindestens von der Vorderseite zu der Hinterseite des absorbierenden Artikels auf der Außenseite in der Breitenrichtung erstreckt.

2. Absorbierender Artikel (1) nach Anspruch 1, wobei
ein Bereich (15) eine Dichte aufweist, die geringer ist als eine Dichte von jedem der Faserklumpen (11), und eine Dichte der synthetischen Fasern (12) um einzelne Faserklumpen gebildet ist.

3. Absorbierender Artikel (1) nach Anspruch 1 oder Anspruch 2, wobei die Biobaumwollfasern (10) mit den synthetischen Fasern (12) nicht thermisch fusioniert sind.

4. Absorbierender Artikel nach einem der Ansprüche 1 bis 3, wobei die Oberfläche von jedem der Faserklumpen (11) mit dem aus Pflanzen stammenden Ölmittel beschichtet ist.

5. Absorbierender Artikel nach einem der Ansprüche 1 bis 4, wobei ein Verhältnis eines Gewichts der Biobaumwollfasern (10) in Bezug auf ein Gesamtgewicht des Vliesstoffs in dem Bereich von 2 % bis 50 % liegt.

6. Absorbierender Artikel nach einem der Ansprüche 1 bis 5, wobei die Biobaumwollfasern (10) eine durchschnittliche Faserlänge aufweisen, die kürzer ist als die durchschnittliche Faserlänge der synthetischen Fasern (12).

7. Absorbierender Artikel nach einem der Ansprüche 1 bis 6, wobei die synthetischen Fasern mit einem aus Pflanzen stammenden Ölmittel beschichtet sind.

8. Absorbierender Artikel nach einem der Ansprüche 1 bis 7, wobei die Vielzahl von Faserklumpen einseitig in Richtung eines Mittelteils des Vliesstoffs in einer Dickenrichtung des Vliesstoffs bereitgestellt ist.

## Revendications

1. Article absorbant (1), comportant :
un tissu non tissé, dans lequel
le tissu non tissé comprend des fibres de coton biologique (10) et des fibres synthétiques thermoplastiques (12),
les fibres de coton biologique forment une pluralité de masses de fibres (11) dans le tissu non tissé, et
les surfaces des fibres de coton biologique ne sont pas revêtues au moyen d'un agent à base d'huile dérivée du pétrole, et dans lequel
l'article absorbant (1) comprend
une direction allant dans le sens longitudinal (L),
une direction allant dans le sens de la largeur (W) orthogonale par rapport à la direction allant dans le sens longitudinal,
une feuille de dessus (3),
une feuille de support (4),
un élément absorbant (7) entre la feuille de dessus et la feuille de support, et
une feuille antifuite imperméable aux liquides (5) mise en œuvre entre la feuille de support et
l'élément absorbant et recouvrant l'élément absorbant,
au moins l'une parmi la feuille de dessus et la feuille de support est réalisée à partir d'un tissu non tissé,
le tissu non tissé a une région qui ne chevauche pas la feuille antifuite (5), et
la région du tissu non tissé qui ne chevauche pas la feuille antifuite s'étend au moins depuis le côté avant jusqu'au côté arrière de l'article absorbant sur la partie extérieure dans la direction allant dans le sens de la largeur.

2. Article absorbant (1) selon la revendication 1, dans lequel
une région (15) ayant une densité inférieure par rapport à une densité de chacune des masses de fibres (11) et par rapport à une densité des fibres synthétiques (12) est formée autour des masses de fibres individuelles.

3. Article absorbant (1) selon la revendication 1 ou la revendication 2, dans lequel les fibres de coton biologique (10) ne sont pas thermiquement fondues sur les fibres synthétiques (12).

4. Article absorbant selon l'une quelconque des revendications 1 à 3, dans lequel la surface de chacune des masses de fibres (11) est revêtue au moyen d'un agent à base d'huile dérivée de plantes.

5. Article absorbant selon l'une quelconque des revendications 1 à 4, dans lequel un rapport entre un poids des fibres de coton biologique (10) et un poids total du tissu non tissé se trouve dans la gamme allant de 2 % à 50 %.

6. Article absorbant selon l'une quelconque des revendications 1 à 5, dans lequel les fibres de coton biologique (10) ont une longueur moyenne de fibre plus courte qu'une longueur moyenne de fibre des fibres synthétiques (12).

7. Article absorbant selon l'une quelconque des revendications 1 à 6, dans lequel les fibres synthétiques sont revêtues au moyen d'un agent à base d'huile dérivée de plantes.

8. Article absorbant selon l'une quelconque des revendications 1 à 7, dans lequel les masses de fibres de la pluralité de masses de fibres sont mises en œuvre de manière sollicitée vers une partie centrale du tissu non tissé dans une direction allant dans le sens de l'épaisseur du tissu non tissé.
